(19) Europäisches Patentamt
European Patent Office
Office européen des brevets

(11) EP 2 902 493 A1

(12) EUROPEAN PATENT APPLICATION

(43) Date of publication:
05.08.2015 Bulletin 2015/32

(51) Int Cl.:
C12P 21/00 (2006.01)

(21) Application number: 14153333.1

(22) Date of filing: 30.01.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Techno-Path (Distribution)
Ballina, Co. Tipperary (IE)

(72) Inventors:
• Thompson, Ben
Sheffield, Yorkshire S118YJ (GB)
• James, David
Sheffield, Yorkshire S119LN (GB)
• Clifford, Jerry
Ballina, Co. Tipperary (IE)

(74) Representative: Purdy, Hugh Barry
PurdyLucey
Intellectual Property
6-7 Harcourt Terrace
D2 Dublin (IE)

(54) **A method of predicting relative fed batch production titer of a panel of clonally-derived producer cells**

(57) A computer implemented method of predicting relative fed batch production titer of a panel of clonal producer cells derived from a single parental host cell population comprises the steps of assaying a level of growth of each clone in the presence and absence of one or more individual chemical cell stressors, comparing the level of growth of each clone in the presence and absence of the or each chemical cell stressor to provide a normalised production titer response for each clone in the or each stressed microenvironment, and inputting a clone-specific production titer response profile comprising the normalised production titer response for each clone in the or each stressed microenvironments into a computational model. The computational model is generated from production titer response profiles obtained from a calibration set of clones with known fed batch production titer, and is configured to output the predicted relative fed batch production titer of the panel of clonal cells.

FIG. 2

EP 2 902 493 A1

**Description**

Introduction

The invention relates to a method of predicting fed batch production titer of a producer cell. In particular, the invention relates to a method of predicting relative fed batch recombinant protein titer of a panel of clonally derived producer cells.

Background to the Invention

Early prediction of the relative ability of CHO cell clonal isolates to serve as manufacturing cell lines is a key component of the cell line development process. Clones apparently productive in static culture can vary substantially in subsequent fed-batch culture performance, both with respect to cell growth and productivity. Current best practice is to measure clone performance early in cell line development using a small-scale, multi-parallel bioreactor system that enables comparison of 50-100 clonal isolates. However, this approach is still both costly and time-consuming.
It is an object of the invention to overcome at least one of the above-referenced problems.

Statements of Invention

The Applicant has discovered that the production titre of producer cell clones in fed batch culture can be accurately predicted by rapid profiling of clone-specific recombinant protein production response to one or more functionally diverse chemical stressors (for example inhibitors and toxins) in static microplate culture. To perform microplate profiling, a fixed number of clonally-derived cells were dispensed into 96-well microplates containing one or more chemical cell stressors separately loaded into discrete wells. Each chemical was soluble in cell growth medium and typically utilized at a concentration pre-determined to inhibit parental CHO cell proliferation in microplates within the $LD_{30}$ to $LD_{80}$ range. After static growth for a growth period (i.e.3 days), supernatant MAb titer was measured by HPLC Protein A assay. Twelve CHO-S clones expressing an IgG1 MAb isolated by limiting dilution cloning were subjected to microplate profiling followed by assessment of optimized fed-batch culture performance (MAb titer [MAb], integral of viable cell concentration to 50% cell viability [$IVCC_{50}$]) in Ehrlenmayer flasks. Using multi-linear modeling, it was demonstrated that clone performance in microplates could predict MAb titer ($r^2 = 0.84$) using clone-specific MAb titer microplate profiles.
In a first aspect, the invention provides a computer-implemented method of predicting production titer of a query producer cell in fed batch culture, the method comprising the steps of:

-   incubating the query producer cell with one or more chemical cell stressors;
-   determining the production titer response of the query producer cell in the presence of the or each chemical cell stressor to generate a query cell-specific production titer response profile;
-   inputting the query cell-specific production titer response profile into a computational model, in which the computational model is generated from production titer response profiles obtained from a calibration set of cells with known fed batch production titre, wherein the computational model is configured to output the predicted fed batch production titre for the cell.

In another aspect, the invention provides a computer-implemented method of predicting relative production titer of a panel of producer cells in fed batch culture, the method comprising the steps of:

-   incubating each producer cell with one or more chemical cell stressors;
-   determining the production titer response of each cell in the presence of the or each chemical cell stressors to generate a cell-specific production titer response profile for each of the panel of producer cells;
-   inputting the cell-specific production titer response profile for each of the panel of producer cells into a computational model, in which the computational model is generated from production titer response profiles obtained from a calibration set of cells with known fed batch production titre, wherein the computational model is configured to output the predicted relative production titer of the panel of producer cells.

When a batch of new clones are generated from a parental cell line, rather than all or a select few being subjected to expensive and time-consuming growth studies in bioreactors, the method of the invention allows a large number of clones to be rapidly assayed for, and ranked according to, predicted fed batch (recombinant protein) production titre in a rapid and high-throughput manner. This informs on which clones to take forward to scale-up/mini-bioreactor studies, for example only the good clones as illustrated in Fig. X below.
The methods of the invention employs data (production titer response profile) from a panel of pre-validated clones (clones of known production titer), and a computational model, ideally a multiple linear computational model, based on this data, to allow prediction of relative fed batch recombinant production titer of a new set of clones based on their rapidly obtained chemical fingerprints.

Preferably, the method comprises an aditional step of ranking the clones according to predicted fed batch production titer.

The method is typically carried out using a microtitre plate, in which each assay is performed in a well of a microtitre plate. Suitably, the growth of each clonal cell is assayed in the well of a multiwell plate. When multiple chemical cell stressors are employed, each clone will be assayed in the presence of single chemical cell stressor.

Typically, the assay involves mixing a sample of a clone with a chemical cell stressor, and incubating the mixture from 1 to 4 days, typically 2 to 4 days, preferably 60-80 hours, and ideally about 3 dyas, and assaying the level of growth of the cells. Suitably, the clone sample is provided at a concentration of from 0.1 to $1.0 \times 10^6$ cells per ml of mixture. Typically, the bioreactor-relevant chemical cell stressor is provided at a concentration of 0.5 to 2 x IC50, ideally about 1 x IC50.

Suitably, the growth of each clone is assayed after a period of incubation of less than 5 days. Ideally, the growth of each clone is assayed after a period of incubation of between 1-4 days, and ideally after 2 and 3 days.

Preferably, the growth of each clone is assayed simultaneously. Typically, the incubation step is carried out in static microplate culture.

In a preferred embodiment, the invention provides a rapid, high-throughput, computer-implemented method of predicting relative production (i.e. monoclonal antibody) titer of a panel of clonal producer cells in fed batch culture, the method comprising the steps of:

- simultaneously incubating each producer cell with a plurality of individual, functionally diverse, chemical cell stressors in static microplate culture for an incubation period of less than four days, in which the chemical cell stressors are selected from at least two of an amino acid transport inhibitors, cell cycle inhibitors, a source of osmotic stress, a source of oxidative stress, an inducer of apoptosis, metabolic effectors, a pH modifier, an inhibitor of glycolysis, and a toxin.;

- after the incubation period determining the production titer response of each cell in the presence of individual each chemical cell stressors to generate a cell-specific production titer response profile for each of the panel of producer cells;

- inputting the cell-specific production titer response profile for each of the panel of producer cells into a computational model, in which the computational model is generated from production titer response profiles obtained from a calibration set of producer cells with known fed batch production titre, wherein the computational model is configured to output the predicted relative fed batch production titer of the panel of producer cells.

The chemical cell stressors are chemicals that cause a reduction in cell growth via one or more of multiple cellular pathways. Suitably, the plurality of chemical cell stressors comprise at least 2, 3, 4, 5, or 6 functionally diverse chemical cell; stresspors, for example at least 3, 4, 5, 6, 7, 8, 9, 10, 12, 14, 16, 18, 20, 21, 22, 23, 24, or 25 different stressors. Typically, the plurality of chemical cell stressors are selected from the group consisting of amino acid transport inhibitors, cell cycle inhibitors, a source of osmotic stress, a source of oxidative stress, an inducer of apoptosis, metabolic effectors, a pH modifier, an inhibitor of glycolysis, and a toxin. These are examples of functionally diverse chemical cell stressors. Typically, the plurality of chemical cell stressors include stressors selected from at least 4, 5, 6 or 7 of the groups consisting of amino acid transport inhibitors, cell cycle inhibitors, a source of osmotic stress, a source of oxidative stress, an inducer of apoptosis, metabolic effectors, a pH modifier, an inhibitor of glycolysis, and a toxin.

In another aspect, the invention provides a microtitre plate comprising at least 24, 48 or 96 wells, and a plurality of chemical cell stressors disposed individually in at least some of the wells. Typically, the plate comprises at least 6, 7, 8, 9, 10, 12, 14, 16, 18, 20, 21, 22, 23, 24, or 25 chemical cell stressors. Suitably, each chemical cell stressor is disposed in at least two or three wells of the plate (i.e. duplicate or triplicate).

Preferably, the plurality of chemical cell stressors are selected from the group of 2-aminobicyclo-(2,2,1)heptane-carboxylic acid (BCH), D-phenylalanine, α-(methylamino)isobutyric acid (MeAIB), Sodium Butyrate (NaBu), cycloheximide, ammonium chloride, Cadmium acetate dihydrate, Cobalt chloride (CoCl2), Sodium Chloride (NaCl), Sodium lactate (Na Lac), Aminotriazole (AMT), Menadione Sodium Bisulphite (MSB), Buthionine Sulfoximine (BSO), Mercaptosuccinic Acid (MS), 2,4,Dinitrophenol (24DNP), Sodium Oxamate, 2-deoxyglucose (2dg), 3-bromopyruvate (3-BrPA), Dichloroacetate (DCA), 6-diazo-5-oxo-1-norleucine (1-don), Valproic acid (Val), Sodium Orthovandate (NaV), citric acid, FK866, lactic acid.

In another aspect, the invention provides a microtitre plate comprising at least 24, 48 or 96 wells, and one or more, genrally a plurality of, chemical cell stressors disposed individually in at least some of the wells, in which when a plurality of different chemical cell stressors is employed the chemical cell stressors comprise at least one chemical cell stressor selected from each of the groups consisting of amino acid transport inhibitors, cell cycle inhibitors, a source of osmotic stress, a source of oxidative stress, an inducer of apoptosis, metabolic effectors, a pH modifier, an inhibitor of glycolysis, and a toxin.

In one embodiment, the invention provides a microtitre plate comprising at least 96 wells, and at least 23 chemical cell stressors disposed individually in wells of the plate, in which the chemical cell stressors consist essentially of 2-aminobicyclo-(2,2,1)heptane-carboxylic acid (BCH), D-phenylalanine, $\alpha$-(methylamino)isobutyric acid (MeAIB), Sodium Butyrate (NaBu), cycloheximide, ammonium chloride, Cadmium acetate dihydrate, Cobalt chloride (CoCl2), Sodium Chloride (NaCl), Sodium lactate (Na Lac), Aminotriazole (AMT), Menadione Sodium Bisulphite (MSB), Buthionine Sulfoximine (BSO), Mercaptosuccinic Acid (MS), 2,4,Dinitrophenol (24DNP), Sodium Oxamate, 2-deoxyglucose (2dg), 3-bromopyruvate (3-BrPA), Dichloroacetate (DCA), 6-diazo-5-oxo-1-norleucine (1-don), Valproic acid (Val), Sodium Orthovandate (NaV), citric acid, FK866, lactic acid.

In another apsect, the invention provides a kit suitable for performing a method of the invention and comprising (a) a microtitre plate of the invention (b) a microtitre plate reader, and (c) a computer program comprising program instructions for (i) receiving from a determination system (for example a HPLC) a production titre value of each clone in the presence and absence of the or each chemical cell stressor, (ii) calculating a normalised production titer value for each clone in the presence of the or each chemical cell stressor, (ii) inputting a clone-specific production titer response profile comprising the normalised production titer value for each clone in the presence of the or each cxhemical cell stressor into a computational model, in which the computational model is generated from production titer response profiles obtained from a calibration set of clones with known production titre values, wherein the computational model is configured to output the predicted fed batch production titre value for each clone (and optionally predict the relative fed batch production titer value of the panel of clonal cells), and (iii) outputting the the predicted fed batch production titre value for each clone (and optionally predict the relative fed batch production titer value of the panel of clonal cells),

In another apsect, the invention provides a kit suitable for performing a method of predicting the relative fed batch production titre of a panel of clonal producer cells derived from a single parental host cell population and comprising (a) a microtitre plate of the invention (b) a microtitre plate reader, and (c) a computer program comprising program instructions for (i) receiving from a suitable machine a production titre value of each clone grown in the presence and absence of one or more chemical cell stressors, (ii) calculating a normalised production titer value for each clone in the presence of the or each chemical cell stressor, (ii) inputting a clone-specific production titer response profile comprising the normalised production titer value for each clone in the presence of the or each chemical cell stressor into a computational model, in which the computational model is generated from production titer response profiles obtained from a calibration set of clones with known production titre values, wherein the computational model is configured to output the predicted fed batch production titre value for each clone and predict the relative fed batch production titer value of the panel of clonal cells, and (iii) outputting the predicted relative fed batch production titer value of the panel of clonal cells.

The determination system employed to determine the production titer of a clone may be any suitable machine, for example a HPLC or mass spectrometer adapted to quantitatively assay the target recombinant protein.

The invention also provides a computer implemented system for performing a method of predicting fed batch production titer of a clonal producer cell derived from a single cell line, the system comprising:

- a determination system for assaying production titer of the clone in the presence and absence of one or more individual chemical cell stressors (typically a plurality of individual chemical cell stressors) to provide a normalised production titer response value for the clone in one or more stressed microenvironments ;
- a computational model adapted to process a clone-specific production titer response profile comprising the normalised production titer response value for the clone in the or each stressed microenvironment, in which the computational model is generated from production titer response profiles obtained from a calibration set of clones with known fed batch production titer response profiles, wherein the computational model is configured to output the predicted fed batch production titer value for the clone, and
- means for outputting the predicted fed batch production titre for the clonal producer cell.

The invention also provides a computer implemented system for performing a method of predicting relative fed batch production titer of a panel of clonal producer cells derived from a single cell line, the system comprising:

- a determination system for assaying production titer of each clone in the presence and absence of a plurality of individual chemical cell stressors to provide a normalised production titer response value for each clone in a plurality of stressed micro environments ;
- a computational model adapted to process a clone-specific production titer response profile comprising the normalised production titer response value for each clone in each of the stressed microenvironments, in which the computational model is generated from production titer response profiles obtained from a calibration set of clones with known fed batch production titer response profiles, wherein the computational model is configured to output the predicted fed batch production titer value for each clone and/or the predicted relative fed batch production titer of the panel of clonal cells, and

- means for outputting (i) the predicted fed batch production titre for one or more clones in the panel, or (ii) the predicted relative fed batch performance of the panel of clonal cells, or (iii) both (i) and (ii).

Typically, the determination system comprises a HPLC, although other systems adapted to quantitatively assay for a target protein, for example a quantitative ELISA, may be employed.

Suitably, the computational model is a multiple linear computational model. Ideally, the multiple linear computational model suitably employs a least squares solution to fit parameters (i.e. levels of cell specific production titer responses) to the observed fed batch production titer.

The invention also provides a computer program which when executed on a computer causes the computer to perform a method of predicting relative fed batch production titer of a panel of clonal cells derived from a single cell line according to the invention.

The invention also relates to a computer program recording medium storing a computer program according to the invention.

Brief Description of the Figures

Figure 1: Maximum monoclonal antibody titre achieved in fed batch culture from a panel of 12 clonal cell lines from a common parental cell line.

Figure 2: The range of product titres in the presence of specific chemical stressors achieved by a panel of 12 clonal cell lines.

Figure 3: A visual representation of the goodness of fit of a multiple linear model to predict maximum MAb titre using chemical specific MAb responses.

Figure 4: An illustration of the ability of the model to predict new data. Here, using the stated model parameters, Max MAb titre was predicted with new data using a "leave one out" cross validation function.

Detailed Description of the Invention

In one aspect, the invention relates to a method of screening a panel of producer cell clones derived from a single cell line to stratify the clones according to fed batch production titer. The method involves incubating a sample of each clone with and without one, and generally a plurality of, individual chemical cell stressors for a period of time, typically of at least 24 hours and up to 4 or 5 days, to obtain a plurality of normalised production titer response values for the clone. These production titer response value(s) provide a specific pattern of clone-specific production titer response that forms a clone-specific growth response profile. A multiple linear computational model is employed to correlate the production titer response profiles with a plurality of production titer response profiles from a calibration set of clones with know fed batch production titer, and predict a fed batch production titer for one or more of the panel of clones. The clones from the panel may then be ranked according to predicted fed batch production titer, which allows producer clones to be chosen for further development.

In this specification, the term "rapid, high-throughput" should be understood to mean that the method can be carried out in four days or less, and in which the incubation of cells during the incubation period may be carried out in the wells of a microtiter plate. The term "production titer" or "production titer response" as applied to a specific producer cell clone refers to the amount of a specific protein, generally a recombinant protein, and ideally a recombinant monoclonal antibody, that the specific producer cell clone generates over a defined time period. The titer may be quantified in absolute or relative terms. Generally titre is referred to as weight of product per volume of culture - grams per litre (g/L) is a common metric (Max titer). Generally, the clones are incubated for a specific time period, for example 2-4 days, and following the incubation period a sample of the supernatant is typically taken and assayed for production titer. Various methods will be apparent to the person skilled in the art for measuring production titer, including HPLC and quantitative ELISA.

In many cases, the production titer employed in the methods of the invention will be normalised production titer response, meaning the difference between the production titer response for the cell when measured in the presence and absence of the chemical cell stressor.

The term "producer cell" refers to a cell that is employed to generate a specific desired protein. Generally, the cell is genetically modifed to include one or multiple copies of a transgene encoding the desired protein which is generally under the control of a specific promotor. Thus, the specific protein is usually a recombinant protein. Producer cells are well known in the art, and include for example Chinese hamster ovary (CHO) cells or baby hamster kidney (BHK) cells. Ideally, the producer cell is a CHO cell. Typically, the producer cell is a monoclonal antibody producer cell.

The term "production titer response profile" refers to the production titer responses for a given clone to one or more chemical cell stressors. In one embodiment, the profile may include a single production titer response for a given clone (the production titer response for the cell in the presence and typically absence of one chemical cell stressor). In other embodiments, the production titer response profile comprises a plurality of production titer responses or a given clone (the production titer responses for the cell in the presence, and optionally absence) of a plurality of individual chemical cell stressor). Generally, the profile is generated using normalized production titer responses (production titer in absence of stressor minus production titer in presence of stressor), so that a normalized production titer response profile is obtained. However, in circumstances where the profile is generated using more than one stressor, it is possible to use profiles generated with production titres from stressed microenvironments only (i.e. without control production titres).

The method of the invention employs rapid profiling of producer cells to predict the production titer of the cells when in fed batch culture. In this specification, the term "fed batch" or "fed batch culture" should be understood to mean extended culture of cells where additional nutrients are added in bolus format at least once post seeding of the cells.

The term "calibration set of clones" refers to a plurality of clones, each having a production titer response fingerprint and a known fed batch performance. Typically, the calibration set of clones includes clones exhibiting a range of fed batch performance abilities from what the end user would consider 'good' performers to what the end user would consider 'bad' performers. Suitably, the calibration set of clones comprises at least 3, 4, 5, 6, 7, 8, 9, 10, 12, 14, 16, 18, 20, 22, 24, or 26 clones

The term "stressed microenvironment" should be understood to mean an environment that includes a chemical cell stressor. Generally, this means that a clone is assayed for growth in a well of microtitre plate in the presence of the chemical cell stressor.

In this specification, the term "rapid" should be understood to mean a method of predicting fed batch performance that takes less than five days, and ideally less than four or three days.

In this specification, the term "high-throughput" should be understood to mean a method in which a large number of samples, for example 20-500, can be assayed simultaneously. In one embodiment, this involves assaying the growth of the clones in a multiwell plate, for example a 48, 96, or 192 well plate.

In this specification, the term "predicted fed batch production titer" as applied to a clonal producer cell should be understood to mean the predicted fed batch production titer of the clone in fed batch culture. The predicted production titer may be quantified in absolute terms, or may be quantified in relative terms, i.e. relative to a clone having known good or bad production titer or relative to a panel of clones.

In this specification, the term "predicting relative production titer" should be understood to mean predicting fed batch production titer of the clones in the panel relative to one another. Thus, in one embodiment, the clones are stratified according to their predicted fed batch production titer. Likewise, the term "predicted relative fed batch production titer of a panel of clonal cells" should be understood to mean the predicted fed batch production titer of the clones in the panel relative to one another. Thus, in one embodiment, the clones are stratified according to their predicted fed batch production titer. In another embodiment, the clones are stratified to pick one or more clones showing best predicted fed bacth production titer clones, one or more clones showing worst predicted fed bacth production titer clones, or both.

In this specification, the term "panel of clonal producer cells" should be understood to mean a panel of clonal producer cell populations derived from a single cell line, and comprising from 2 to 500 or more clonal producer cell populations. Methods for generating panels of clonal producer cells are well known to a person skilled in the art, and described in Production of recombinant protein therapeutics in cultivated mammalian cells (2004) ,Wurm, Florian M, New York, NY, Nature Biotechnology 22 (2004), S. 1393-1398. Typically, the panel of clonal producer cell populations include from 10-500, 20-500, 30-500, 40-500, 50-500, 60-500, 70-500, 80-500, 90-500 or 100-500 clonal cell populations. Typically, the panel of clonal producer cell populations include from 100-500, 100-400, 150-400, 150-350 clonal cell populations.

In this specification, the term "computational model" typically refers to a multiple linear computational model. Ideally, the multiple linear computational model employs a least squares solution to fit parameters (i.e. levels of cell specific production titer responses) to the observed fed batch production titer.

In this specification, the term "chemical cell stressor" refers to a chemical that causes a reduction in cell growth via one or more of multiple cellular pathways. Suitably, the plurality of chemical cell stressors comprise at least 3, 4, 5, 6, 7, 8, 9, 10, 12, 14, 16, 18, 20, 21, 22, 23, 24, or 25 stressors. Typically, the plurality of chemical cell stressors are selected from the group consisting of amino acid transport inhibitors, cell cycle inhibitors, a source of osmotic stress, a source of oxidative stress, an inducer of apoptosis, metabolic effectors, a pH modifier, an inhibitor of glycolysis, and a toxin. Typically, the plurality of chemical cell stressors include stressors selected from at least 4, 5, 6 or 7 of the groups consisting of amino acid transport inhibitors, cell cycle inhibitors, a source of osmotic stress, a pH modifier, a source of oxidative stress, an inducer of apoptosis, metabolic effectors, an inhibitor of glycolysis, and a toxin. Preferably, the chemical cell stressor is selected from the group of 2-aminobicyclo-(2,2,1)heptane-carboxylic acid (BCH), D-phenyla-lanine, $\alpha$-(methylamino)isobutyric acid (MeAIB), Sodium Butyrate (NaBu), cycloheximide, ammonium chloride, Cadmium acetate dihydrate, Cobalt chloride (CoCl2), Sodium Chloride (NaCl), Sodium lactate (Na Lac), Aminotriazole (AMT), Menadione Sodium Bisulphite (MSB), Buthionine Sulfoximine (BSO), Mercaptosuccinic Acid (MS), 2,4,Dinitrophenol (24DNP), Sodium Oxamate, 2-deoxyglucose (2dg), 3-bromopyruvate (3-BrPA), Dichloroacetate (DCA), 6-diazo-5-oxo-1-norleucine (1-don), Valproic acid (Val), Sodium Orthovandate (NaV), citric acid, FK866, lactic acid.

Exprimental

*Cell culture:*

Clones used were CHO-S derived clones from a monoclonal antibody producing parental clone (clone 38) provided by Cobra biologics (Keele, UK). These are henceforth referred to as "PM-CHO". PM-CHO Cells were cultured in CD-CHO media (Invitrogen, Paisley, UK), 8mM L-Glutamine (Invitrogen, Paisley, UK), 1% HT supplement (Invitrogen, Paisley, UK) 12.5 $\mu$g/ml puromycin (Invitrogen, Paisley, UK). Cells were routinely sub-cultured on an alternating 3 / 4 day regime.

*Fed batch studies:*

Fed batch studies were performed in 60 ml volumes in shake flasks using commercially available feed and media. Feed was added in 6 ml boluses on days 4,6,8 and 10. Samples were taken daily and analysed for cell growth and monoclonal antibody titre.

*Determination of Monoclonal antibody titre:*

A HPLC method was employed using a "Biomonolith" protein A column (Aglient, Workingham, UK). Monoclonal antibody was quantified according to area under UV absorption peak in the eluent. This was demonstrated to be linearly proportional to antibody concentration in the sample.

*Method of performing invention:*

From a detailed literature survey, a variety of chemicals which were anticipated to be informative of fed batch performance were identified. These were:

> 2-aminobicyclo-(2,2,1)heptane-carboxylic acid (BCH)
> D-phenylalanine -(D-Phe)
> $\alpha$-(methylamino)isobutyricacid (MeAIB)
> Sodium Butyrate (NaBu)
> Cycloheximide
> Ammonium chloride
> Cadmium acetate dihydrate
> Cobalt chloride (CoCl2)
> Sodium Chloride (NaCl)
> Sodium lactate (Na Lac)
> Aminotriazole (AMT)
> Menadione Sodium Bisulphite (MSB)
> Buthionine Sulfoximine (BSO)
> Mercaptosuccinic Acid (MS)
> 2,4,Dinitrophenol (24DNP)
> Sodium Oxamate
> 2-deoxyglucose (2dg)
> 3-bromopyruvate (3-BrPA)
> Dichloroacetate (DCA)
> 6-diazo-5-oxo-1-norleucine (1-don)
> Valproic acid (Val)
> Sodium Orthovandate (NaV)
> Citric acid

FK866

Lactic acid

Using an 'in house' parental cell line, inhibitory dose 50 (IC50) concentrations of the above chemicals were identified through growth studies. Here IV50 is defined as the concentration of the chemical, in question, which inhibits normal cell growth by 50% over a 3 day period. Once IC50s were established, 96 well plates were set up containing the above selection of chemicals at the IC50 concentration and including control wells which contained only cell growth media.

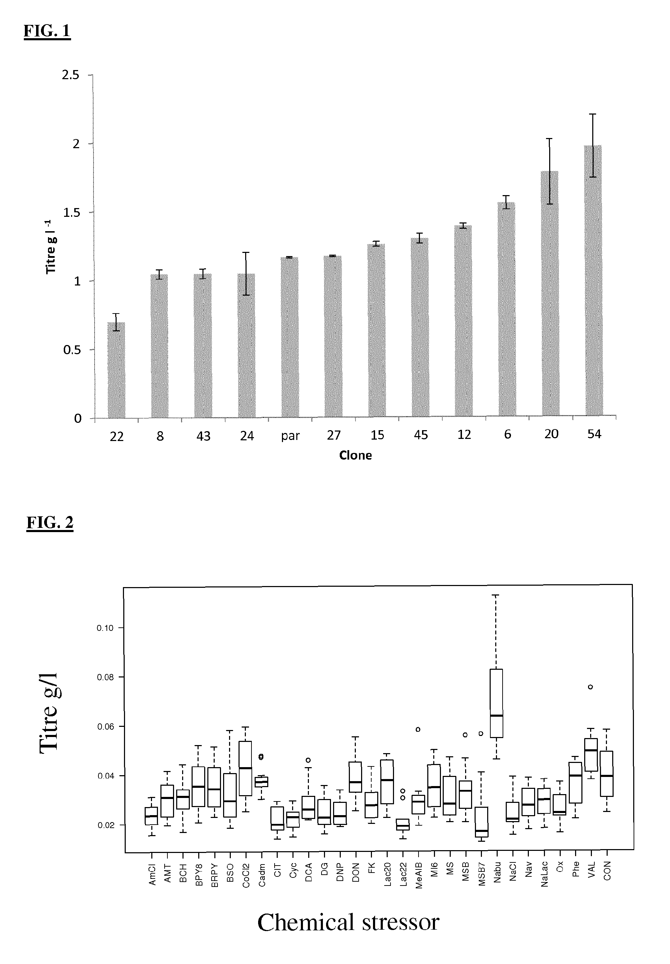

A panel of size 12 of monoclonal antibody producing clonal cell lines was generated in house by a limiting dilution cloning method using the PM parental cell line. These clones were subjected to fed batch studies to asses their fed batch product production ability. Here this is defined as the maximum volumetric product titre achieved during the fed batch run. The range of maximum titres achieved during fed batch by the clonal panel is illustrated in figure 1.

Each clonal cell line was grown in a 96 well plate containing the above mentioned chemicals for 3 days. After this period the level of chemical specific product titre in the plates (i.e. MAb titre in the presence and absence of chemicals as mentioned above - normalized production titer) was measured using the "HPLC" assay as described above. This allowed a chemical specific product production fingerprint to be identified for each clonal cell line (referred to above as a clone specific production titer response profile). Here a product production fingerprint is defined as the plurality of product production levels in each chemical microenvironment.

The range of chemical specific productivities for the panel of clones is illustrated in Fig. 2.

Using information from these chemical specific product production fingerprints, a multiple linear model was built using responses to individual chemicals as parameters.

A multiple linear model is defined as that satisfying the equation.

$$\hat{\beta} = (\mathbf{X}^{\mathrm{T}}\mathbf{X})^{-1}\mathbf{X}^{\mathrm{T}}y = \left(\tfrac{1}{n}\sum x_i x_i^{\mathrm{T}}\right)^{-1}\left(\tfrac{1}{n}\sum x_i y_i\right).$$

Where X represents the matrix containing appropriate data from the explanatory variables and Y is the vector of dependent (or response) variables. This is essentially finding a least squares solution to fit parameters (i.e. levels of chemical specific product production) to the observed fed batch performance (here defined as Max MAb titre).

An example of a multiple linear model built from the plurality of clone microenvironments is

$$\mathrm{Max.MAb.Titre} = 37.78*(\mathrm{Control\ plate\ titre}) - 22.4150*(\mathrm{MeIAB\ plate\ titre}) + 0.4711$$

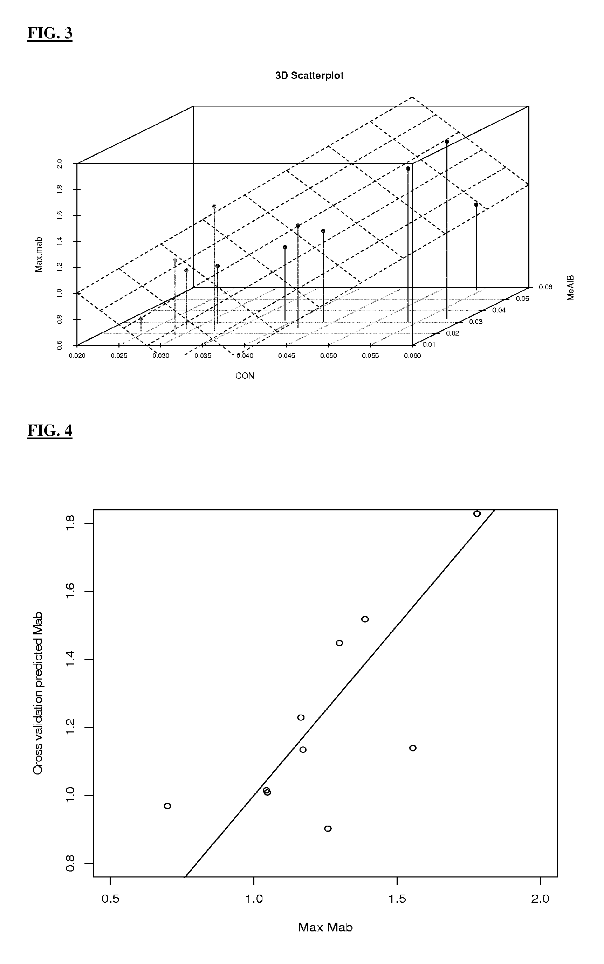

Where Max.Mab.Titre is the maximum monoclonal antibody titre achieved in fed batch culture. A graph illustrating the goodness of model fit using the above parameters is shown in Fig. 3. The $R^2$ value for this model is 0.84. This can be interpreted that 84% of the behavior of the data can be explained by the model.

By using bootstrap cross validation techniques it can be shown that a model built in the above way can be predictive of future data, i.e. data not used in the model. Fig. 4 illustrates the predictive ability of the modeling approach to build predictive models. With this model, a multiple $R^2$ of 0.68 was achieved for predicting new data.

This model, build on a set of clones with known fed batch performance can use the 'fingerprint' of new clones and be able to predict their relative fed batch performance. Initially a model is generated using a panel of clones with known fed batch performance (in the above example 12 were used but there is no limitation to how many can be used - However the complexity of the model built is limited by the number of clones used). Subsequently when a large panel of clones is derived in the process of cell line development, these will be "screened" in the multiple microenvironment plates, and using the model generated from clones with known performance characteristics, it is possible to predict Max MAb titre, rank the clones or at least give information as to the likelihood of their future fed batch performance capabilities therefore aiding the process of selecting which clones are taken forward to the next stage of clones screening (i.e. small scale fed batch studies).

The invention is not limited to the embodiments hereinbefore described which may be varied in construction and detail without departing from the spirit of the inventon.

**Claims**

1. A rapid, high-throughput, computer-implemented method of predicting relative production titer of a panel of clonal producer cells in fed batch culture, the method comprising the steps of:

   - simultaneously incubating each producer cell with at least one chemical cell stressor for an incubation period of less than four days;
   - after the incubation period determining the production titer response of each cell in the presence and absence of the at least one chemical cell stressor to generate a cell-specific production titer response profile for each of the panel of producer cells;
   - inputting the cell-specific production titer response profile for each of the panel of producer cells into a computational model, in which the computational model is generated from production titer response profiles obtained from a calibration set of producer cells with known fed batch production titre, wherein the computational model is configured to output the predicted relative fed batch production titer of the panel of producer cells.

2. A method according to Claim 1 in which each producer cell is incubated with a plurality of individual chemical cell stressors, wherein the cell-specific production titer response profile for each of the panel of producer cells comprises a plurality of production titer responses.

3. A method according to Claim 1 or 2 in which the production titer responses comprise normalized production titer responses.

4. A method according to any preceding Claim, in which the production titer is the production titer of a recombinant protein, wherein the producer cell comprises a transgene encoding the recombinant protein.

5. A method according to Claim 4, wherein the recombinant protein is a monoclonal antibody, or a fragment thereof.

6. A method according to any preceding Claim, in which the panel of clonal producer cells are CHO cells.

7. A method according to any preceding Claim in which each producer cell is incubated with the or each individual chemical cell stressor in the wells of a microtitre plate in static microplate culture.

8. A method according to any preceding Claim in which the or each chemical cell stressor is employed at a concentration that is pre-determined to inhibit proliferation of the clonal producer cells during incubation within the LD30 to LD80 range.

9. A method according to any preceding Claim in which the plurality of chemical cell stressors comprise at least two of: amino acid transport inhibitors; cell cycle inhibitors; a source of osmotic stress; a source of oxidative stress; an inducer of apoptosis; an inhibitor of glycolysis; and a toxin.

10. A method according to any preceding Claim in which the plurality of chemical cell stressors comprises an animo acid transport inhibitor.

11. A rapid, high-throughput, computer-implemented method of predicting relative production titer of a panel of clonal producer cells in fed batch culture according to any preceding Claim, the method comprising the steps of:

   - simultaneously incubating each producer cell with a plurality of individual, functionally diverse, chemical cell stressors in static microplate culture for an incubation period of less than four days, in which the chemical cell stressors are selected from at least two of an amino acid transport inhibitors, cell cycle inhibitors, a source of osmotic stress, a source of oxidative stress, an inducer of apoptosis, metabolic effectors, a pH modifier, an inhibitor of glycolysis, and a toxin.;
   - after the incubation period determining the production titer response of each cell in the presence of each individual chemical cell stressors to generate a cell-specific production titer response profile for each of the panel of clonal producer cells;
   - inputting the cell-specific production titer response profile for each of the panel of producer cells into a computational model, in which the computational model is generated from production titer response profiles obtained from a calibration set of producer cells with known fed batch production titre, wherein the computational model is configured to output the predicted relative fed batch production titer of the panel of producer cells.

12. A computer implemented system for performing a method of predicting relative production titer of a panel of clonal producer cells in fed batch culture, the system comprising:

> - a determination system for assaying production titer of each clone in the presence and absence of a plurality of individual chemical cell stressors to provide a normalised production titer response value for each clone in a plurality of stressed micro environments ;
> - a computational model adapted to process a clone-specific production titer response profile comprising the normalised production titer response value for each clone in each of the stressed microenvironments, in which the computational model is generated from production titer response profiles obtained from a calibration set of clones with known fed batch production titer response profiles, wherein the computational model is configured to output the predicted fed batch production titer value for each clone and/or the predicted relative fed batch production titer of the panel of clonal cells, and
> - means for outputting (i) the predicted fed batch production titre for one or more clones in the panel, or (ii) the predicted relative fed batch performance of the panel of clonal cells, or (iii) both (i) and (ii).

13. A system as claimed in Claim 12, in which the determination system comprises a HPLC.

14. A method according to any of Claims 1 to 11, or a system according to Claim 12 or 13, in which the computational model is a multiple linear computational model.

**FIG. 1**

**FIG. 2**

**FIG. 3**

3D Scatterplot

**FIG. 4**

Europäisches
Patentamt

European
Patent Office

Office européen
des brevets

# EUROPEAN SEARCH REPORT

Application Number

EP 14 15 3333

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RACHEL LEGMANN ET AL: "A Strategy for clone selection under different production conditions", BIOTECHNOLOGY PROGRESS, vol. 27, no. 3, 29 May 2011 (2011-05-29), pages 757-765, XP055084189, ISSN: 8756-7938, DOI: 10.1002/btpr.577 * the whole doc, in particular p.758, right col. 3rd paragraph and table I, p. 761, left col. 2nd paragraph-right col., fig. 4 and 5. * | 1-14 | INV. C12P21/00 |
| A | JIANLIN XU ET AL: "Galactose can be an inducer for production of therapeutic proteins by auto-induction usingBL21 strains", PROTEIN EXPRESSION AND PURIFICATION, vol. 83, no. 1, 18 June 2005 (2005-06-18), pages 30-36, XP028413544, ISSN: 1046-5928, DOI: 10.1016/J.PEP.2012.02.014 [retrieved on 2012-03-08] * the whole document * | 1-14 | |
| | -/-- | | TECHNICAL FIELDS SEARCHED (IPC) C12P G06F |

The present search report has been drawn up for all claims

| Place of search | Date of completion of the search | Examiner |
|---|---|---|
| Munich | 13 June 2014 | Lüdemann, Susanna |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
   after the filing date
D : document cited in the application
L : document cited for other reasons

    .............................................................................

& : member of the same patent family, corresponding
    document

EPO FORM 1503 03.82 (P04C01)

**EUROPEAN SEARCH REPORT**

Application Number

EP 14 15 3333

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HAIMANTI DORAI ET AL: "Combining high-throughput screening of caspase activity with anti-apoptosis genes for development of robust CHO production cell lines", BIOTECHNOLOGY PROGRESS, vol. 26, no. 5, 24 September 2010 (2010-09-24), pages 1367-1381, XP055123365, ISSN: 8756-7938, DOI: 10.1002/btpr.426 * whole doc, in particular title, abstract, p. 1470, left col. last paragraph, p. 1371, left col., third para and fig. 8, p. 1374, right col. last para - p. 1375, left col. and fig. 7. * | 1-14 | |
| A | DA CRUZ MELEIRO L A ET AL: "Non-Linear Multivariable Predictive Control of an Alcoholic Fermentation Process Using Functional Link Networks", BRAZILIAN ARCHIVES OF BIOLOGY AND TECHNOLOGY, INSTITUTO DE TECNOLOGIA DO PARANA, BR, vol. 48, no. Special, 1 June 2005 (2005-06-01), pages 7-18, XP002490882, ISSN: 1516-8913 * the whole document * | 1-14 | TECHNICAL FIELDS SEARCHED (IPC) |

The present search report has been drawn up for all claims

| Place of search | Date of completion of the search | Examiner |
|---|---|---|
| Munich | 13 June 2014 | Lüdemann, Susanna |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WURM, FLORIAN M.** *Nature Biotechnology,* 2004, vol. 22, 1393-1398